# NEUE EUROPÄISCHE PATENTSCHRIFT

(11) **EP 2 410 964 B2**
(45) Veröffentlichungstag und Bekanntmachung des Hinweises auf die Entscheidung über den Einspruch: **18.03.2020**
(45) Hinweis auf die Patenterteilung: 15.05.2013
(21) Anmeldenummer: 10710554.6
(22) Anmeldetag: 24.03.2010
(51) Int. Cl.: A61F 13/15, A61F 13/56

(54) **ABSORBIERENDE INKONTINENZWEGWERFWINDEL MIT SEITENABSCHNITTEN**
ABSORBENT DISPOSABLE INCONTINENCE PANTS COMPRISING SIDE SECTIONS
COUCHE POUR INCONTINENCE ABSORBANTE ET JETABLE, COMPORTANT DES PARTIES LATÉRALES

(30) Priorität: 26.03.2009 DE 102009015041
(43) Veröffentlichungstag der Anmeldung: 01.02.2012
(73) Patentinhaber: Paul Hartmann AG, 89522 Heidenheim (DE)
(72) Erfinder: GAUSE, Enno, 89522 Heidenheim (DE); KESSELMEIER, Rüdiger, 89542 Herbrechtingen (DE); MALOWANIEC, Krzysztof-Daniel, 89522 Heidenheim (DE); SWEREV, Maximilian, 86169 Augsburg (DE)
(74) Vertreter: DREISS Patentanwälte PartG mbB
(86) Internationale Anmeldenummer: PCT/EP2010/001819
(87) Internationale Veröffentlichungsnummer: WO 2010/108660

(56) Entgegenhaltungen:
- EP-A1- 1 719 484
- EP-A1- 1 941 853
- EP-A1- 2 020 215
- WO-A1-2009/002235
- WO-A1-2009/015746
- WO-A1-2009/015746
- WO-A1-2010/028786
- WO-A1-2010/051934
- WO-A2-2007/043027
- JP-A- 4 261 655
- US-A- 6 004 306
- US-A1- 2003 226 862
- US-A1- 2006 282 053
- US-A1- 2008 208 152

## Beschreibung

Die Erfindung betrifft einen absorbierenden Inkontinenzartikel des offenen Typs mit einem Hauptteil, bestehend aus einem Vorderbereich, einem Rückenbereich und einem in Längsrichtung dazwischen liegenden zwischen den Beinen eines Benutzers zu liegen kommenden Schrittbereich, wobei der Hauptteil einen Saugkörper umfasst, und mit an den Rückenbereich und/oder an den Vorderbereich beidseits angefügten, voneinander separaten mit Verschlussmitteln versehenen Seitenabschnitten, welche sich in Querrichtung über seitliche Längsränder des Hauptteils hinaus erstrecken und den Vorderbereich und den Rückenbereich im angelegten Zustand des Artikels miteinander verbinden.

Derartige Inkontinenzartikel sind bekannt und beispielsweise in WO 2005/102241 A1 beschrieben. Die Seitenabschnitte, die mitunter auch als Ohren bezeichnet werden, werden vorzugsweise im Cut & Place-Verfahren direkt an den Hauptteil, das Chassis des Hygieneartikels angefügt. Diese Fertigungstechnologie erlaubt es, die Seitenabschnitte aus einem anderen Rohmaterial zu fertigen als den mittleren Hauptteil des Hygieneartikels. Beispielsweise können die Seitenabschnitte luftdurchlässig ausgeführt werden, wohingegen der mittlere Hauptteil im Wesentlichen feuchtigkeitsundurchlässig ausgebildet werden kann.

Die aus der Fertigungssicht effizienteste und einfachste sowie kostengünstigste Form der Seitenabschnitte ist die rechteckige Form. Sie erlaubt bei der Herstellung den Transport der die Seitenabschnitte bildenden Materialien in Form einer endlosen Flachmaterialbahn, von der dann die Seitenabschnitte quer zur Maschinenrichtung abgetrennt werden. Ein Schnittabfall ist hier praktisch nicht gegeben.

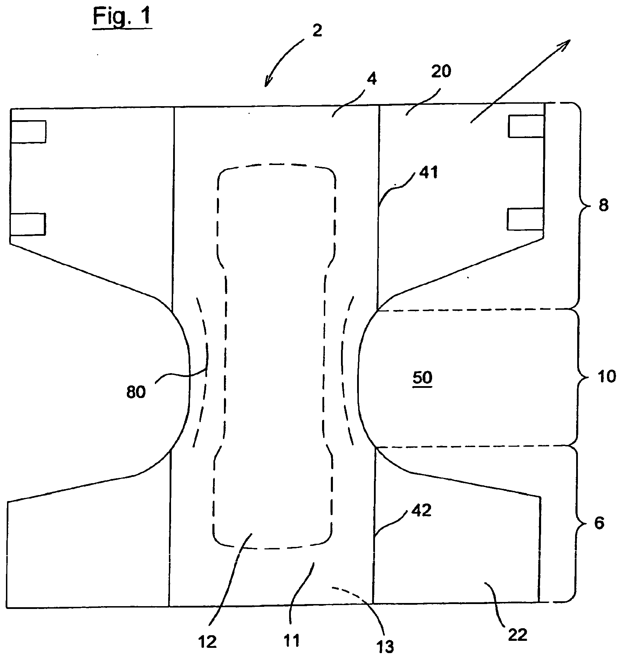

Es hat sich jedoch gezeigt, dass insbesondere bei der Ausbildung der Seitenabschnitte in der ansonsten vorteilhaften Rechteckform beim Anlegen und Tragen des Hygieneartikels mitunter das Problem besteht, dass die angefügten Seitenabschnitte im Bereich der seitlichen Längsränder des Hauptteils einreißen können. Es hat sich nämlich gezeigt, dass Anwender beim Anlegen des Hygieneartikels geneigt sind, einen zur Quer- und Längsrichtung des Hygieneartikels schrägen Zug auf die Seitenabschnitte auszuüben, was in Figur 1 mit einem schräg nach oben geneigten Pfeil angedeutet ist. Es kann solchenfalls vorkommen, dass Seitenabschnitte entlang der seitlichen Längsränder des Hauptteils einreißen, wobei der Riss ausgehend von dem dem Schrittbereich zugewandten Querrand des Seitenabschnitts ausgeht. Bislang wurde versucht, die Anfügung von derartigen Seitenabschnitten an den Hauptteil von Hygieneartikeln durch ein optimiertes Fügemuster zu verbessern, gemäß WO 2004/017882 A2 und WO 02/17843 A2.

Ein weiterer aus dem Stand der Technik bekannter Vorschlag ist, die Seitenabschnitte mit einem Verstärkungsmittel zu versehen, welches in Querrichtung betrachtet schmäler ausgebildet ist als ein jeweiliger Seitenabschnitt und welches wenigstens in einem den seitlichen Längsrand des Hauptteils überbrückenden Bereich vorgesehen ist, also sowohl einen seitlichen Längsrandbereich des Hauptteil als auch einen Teil des Seitenabschnitts in Querrichtung überfängt (DE102006050971A1).

Der vorliegenden Erfindung liegt die Aufgabe zugrunde, das vorstehend geschilderte Problem noch effektiver zu lösen, also absorbierende Inkontinenzartikel mit vorn und hinten jeweils zwei seitlich angestückten und angefügten Seitenabschnitten zu schaffen, bei denen das Ausreißverhalten der Seitenabschnitte signifikant verbessert ist.

Diese Aufgabe wird durch einen absorbierenden Inkontinenzartikelmit den Merkmalen des Anspruchs 1 gelöst. Hierbei werden R, Fm und C wie weiter unten beschrieben ermittelt.

Wie unten noch näher erläutert und nachgewiesen werden wird, hat bereits die Konturierung der Seitenabschnitte zur Folge, dass die Seitenabschnittsausreißfestigkeit signifikant erhöht wird, da bei der konturierten Form der Seitenrand des Hauptteils weniger geeignet ist, eine Art Abreißkante für die Seitenabschnitte zu bilden. Außerdem wird die Zugkraft, die beim Anlegen der Windel über die beinöffnungsnahen Verschlussmittel in den Seitenabschnitt eingeleitet wird, auf eine größere Fläche verteilt, so dass die auf den kritischen Punkt einwirkende resultierende Kraft deutlich vermindert ist. Unter dem kritischen Punkt wird der Punkt verstanden, an dem der untere Rand des Seitenabschnitts den hinteren Seitenrand des Hauptteils trifft.

Bereits ein sehr geringes Längen- zu Breitenverhältnis R = A/B der Seitenabschnittsbeinöffnungsbereiche ist hinreichend, um diesbezüglich einen signifikant positiven Effekt auszuüben. Ein Wert R von größer 0,4 würde der Passform sowie dem Anlegekomfort der Inkontinenzwegwerfwindel hingegen abträglich sein und außerdem die Menge des zu verwerfenden Schnittabfalls erhöhen.

Es wurde weiterhin erkannt, dass die Weiterreißfestigkeit des die Seitenabschnitte bildenden Materials von lediglich mindestens 4.0 N kombiniert mit der erfindungsgemäßen Konturierung der Seitenabschnitte gestattet, die Verschlussmittel sehr dicht, das heißt höchstens 5 cm davon beabstandet an den unteren Rand der hinteren Seitenabschnitte zu positionieren. Es wurde zwar erkannt, dass die oben beschriebene Gefahr des Einreißens der Seitenabschnitte entlang der seitlichen Längsränder des Hauptteils mit der Entfernung der Verschlussmittel von dem unteren Rand der Seitenabschnitte abnimmt; gleichzeitig ginge damit jedoch ein erheblicher Verlust an Komfort einher, die Windel mit den sehr weit in Windellängs- und -querrichtung ausladenden Seitenabschnitten passgenau an einer Person anzulegen. Das passgenaue Anlegen der Windel lässt sich mit sehr dicht an dem unteren Rand der Seitenabschnitte positionierten Verschlussmittel wesentlich einfacher bewerkstelligen, da hierdurch über die Verschlussmittel ein Zug auf nahezu die gesamte Seitenabschnittslänge ausgeübt werden kann. Überraschenderweise löst die Erfindung diesen noch nicht bekannten Zielkonflikt durch die im Anspruch 1 spezifizierte Merkmalskombination. Die vorliegende Erfindung ermöglicht überdies, ein hohes Maß an Flexibilität bei der Auswahl der Materialien für die hinteren Seitenabschnitte, da hierdurch auch Materialien mit hohem Tragekomfort jedoch geringer Zugfestigkeit (tensile strength), eingesetzt werden können.

Zwar sind gattungsgemäße Inkontinenzwindeln mit schräg zur Längsrichtung verlaufenden oder kurvenförmig ausgebildeten Seitenabschnitten bereits bekannt (W02009/015746A1). Mit der WO2009/015746A1 ist hingegen weder das der vorliegenden Erfindung zugrunde liegende Problem noch dessen Lösung offenbart.

Vorteilhafterweise werden die schräg zur Längsrichtung verlaufend oder kurvenförmig ausgebildeten Seitenabschnittsbeinöffnungsbereiche durch einen kontinuierlich oder quasikontinuierlich geführten Abtrennvorgang, insbesondere durch einen Schnitt oder eine Stanzung, gebildet, so dass ein stetiger durchgehender Rand gebildet wird. Die Bahn des Abtrennvorgangs erfasst dabei den hinteren Seitenabschnitt, den Hauptteil und den vorderen Seitenabschnitt. Die Beinöffnungsbereiche sind somit ausschließlich durch Schnitt-oder Trennkanten des einzigen, kontinuierlichen oder quasikontinuierlichen Abtrennvorgangs gebildet, was natürlich auch eine wirtschaftliche Herstellbarkeit der Inkontinenzwegwerfwindel impliziert und unerwünschte Kanten vermieden werden.

Bei diesem Abtrennvorgang muss in jedem Fall der von dem hinterem Seitenabschnitt, Hauptteil und vorderem Seitenabschnitt gebildete zusammenhängende Schnittabfall aus dem Prozess abgeführt werden. Dies erfolgt vorteilhaft nach dem in DE102008056220 beschriebenen Verfahren. Diesbezüglich wird vollumfänglich und ausdrücklich auf den Offenbarungsgehalt der DE102008056220 Bezug genommen.

Vorzugsweise beträgt die größte Längserstreckung I₁, I₂ des vom hinteren und/oder vorderen Seitenabschnitt abgetrennten Bereichs 20-180 mm, insbesondere 30-100 mm. Die Erstreckung I₃ des vom Hauptteil abgetrennten Bereichs des Schnittabfalls in Längsrichtung der Inkontinenzwegwerfwindel beträgt vorzugsweise 110 bis 500 mm, insbesondere 200 bis 450 mm; hingegen ist die größte Quererstreckung I₄ dieses vom Hauptteil abgetrennten Bereichs eher gering, sie beträgt vorzugsweise 5 bis 100, insbesondere 8 bis 70 und weiter insbesondere 10 bis 60 mm.

Die Erstreckung I₅ des Schnittabfalls in der genannten Querrichtung beträgt insbesondere 150 bis 350 mm und weiter insbesondere 190 bis 300 mm.

In weiterer Ausbildung der Erfindung wird vorgeschlagen, dass das Längen- zu Breitenverhältnis R = A/B der hinteren Seitenabschnittsbeinöffnungsbereiche mindestens 0,15, vorzugsweise 0,18-0,35, besonders bevorzugt 0,20-0,32 beträgt.

Nach einem weiteren Erfindungsgedanken beträgt der Abstand C der Verschlussmittel von dem unteren, dem Schritt zugewandten Rand der hinteren Seitenabschnitte höchstens 4,0 cm, vorzugsweise höchstens 3,5 cm, besonders bevorzugt höchstens 3 cm und ganz besonders mindestens 0,5 cm.

Das Flächengewicht des die hinteren Seitenabschnitte bildenden Materials sollte vorzugsweise 14-40 g/m² insbesondere 16-30 g/m² und ganz besonders 17-28 g/m² betragen.

Die Weiterreißfestigkeit des die hinteren Seitenabschnitte bildenden Materials gemessen und ermittelt als mittlere Kraft Fm wie unten näher beschrieben beträgt vorzugsweise mindestens 5,0 N, besonders bevorzugt mindestens 6,0 N und ganz besonders bevorzugt mindestens 6,5 N, vorzugsweise jedoch höchstens 10,0 N.

Die Weiterreißfestigkeit des die hinteren Seitenabschnitte bildenden Materials gemessen und ermittelt als Mittelwert der Kraftspitzen Fm.sp wie unten näher beschrieben beträgt vorzugsweise mindestens 5,5 N, besonders bevorzugt mindestens 6,0 N, ganz besonders bevorzugt mindestens 6,5 N und insbesondere mindestens 7,0 N, vorzugsweise jedoch höchstens 12 N.

Die Weiterreißfestigkeit des die hinteren Seitenabschnitte bildenden Materials gemessen und ermittelt als maximale Spitzenkraft Fsp wie unten näher beschrieben beträgt vorzugsweise mindestens 5,5 N, besonders bevorzugt mindestens 6,0 N, ganz besonders bevorzugt mindestens 6,5 N und insbesondere mindestens 7,0 N, vorzugsweise jedoch höchstens 12 N.

In weiterer Weiterbildung der Erfindung hat es sich als vorteilhaft erwiesen, auch die vorderen Seitenabschnitt mit einem Länge-zu-Breitenverhältnis R und/oder mit einem Flächengewicht wie oben für die hinteren Seitenabschnitte beschrieben zu versehen. Vorzugsweise weisen außerdem auch die vorderen Seitenabschnitte eine Weiterreißfestigkeit Fm und/oder Fm,sp und/oder Fsp wie für die hinteren Seitenabschnitte beschrieben auf.

Nach einer bevorzugten Ausführungsform der Erfindung verlaufen ein innerer Rand und äußerer Rand der vorderen und/oder hinteren Seitenabschnitte parallel zueinander. Weiter bevorzugt verläuft der innere Rand zumindest abschnittsweise parallel zu einer Längsrichtung der Inkontinenzwegwerfwindel. Vorzugsweise weist der innere Rand eine größere Erstreckung D in der Längsrichtung auf als der äußere Rand.

Es hat sich weiter als vorteilhaft erwiesen, die vorderen und/oder hinteren Seitenabschnitte aus einem Vliesstoffmaterial zu bilden. Geeignet sind insbesondere alle Vliesstoffmaterialien, die zumindest eine Rezepturkomponente auf Basis eines thermoplastischen Polymers enthalten. Die Vliesstoffe können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, Spinnvliese, wasserstrahlvernadelte Vliese, SM-Vliese, SMS-Vliese, SMMS-Vliese oder auch Laminate aus einer oder mehreren dieser Vliesarten, wobei S für Spunbond- und M für Meltblown-Vliesschichten steht. Denkbar und vorteilhaft ist des Weiteren, die vorderen und/oder hinteren Seitenabschnitte aus einem Vliesstoff-Folienlaminat zu bilden, Solchenfalls würde die Folienkomponente außen zu liegen kommen und die Vliesstoffkomponente innen, um körperzugewandt eine weiche Oberfläche zu gewährleisten. In Weiterbildung dieses Erfindungsgedankens ist vorteilhaft, die vorderen und/oder hinteren Seitenabschnitte aus einem Vliesstoff-Folie-Vliesstofflaminat zu bilden, bei dem eine Folienkomponente sandwichartig zwischen zwei Vlieskomponenten angeordnet ist.

Des Weiteren erweist es sich als vorteilhaft, dass seitlich neben den Längsrändern des Saugkörpers erste elastische Elemente mit einer Komponente in Längsrichtung an den Hauptteil angefügt sind. Diese elastischen Elemente können exakt, das heißt geradlinig in Längsrichtung verlaufen oder besonders vorteilhaft auch einer gewissen Konturierung entlang der Beinöffnungen folgend vorgesehen werden. Die elastischen Elemente nehmen solchenfalls einen gekrümmten Verlauf entlang der Beinöffnung. In besonderer Weiterbildung dieses Erfindungsgedankens ist vorgesehen, dass die elastischen Elemente sich nicht in die Seitenabschnitte hinein erstrecken, sondern auf eine Positionierung innerhalb des Hauptteils limitiert sind. Des Weiteren können in der ersten Längsrichtung erstreckte zweite elastische Elemente, insbesondere in Form von so genannten und an sich zum Beispiel auch aus EP0263720A1 bekannten aufstehenden Cuff-Elementen, an die Windelhauptteilbahn angefügt werden. Diese vorzugsweise aufstehenden zweiten elastischen Elemente flankieren gewissermaßen ein Zentrum des Windelhauptteils oder Saugkörpers; sie können im Bereich der Saugkörperränder, innerhalb der Saugkörperränder oder außerhalb der Saugkörperränder vorgesehen werden. Sie bilden einen Seitenauslaufschutz der Inkontinenzwegwerfwindel.

In Weiterbildung der Erfindung ist vorgesehen, dass die Verschlussmittel zum bestimmungsgemäßen Festlegen der Inkontinenzwegwerfwindel an den Körper eines Menschen zumindest bereichsweise sowohl an der Außenseite des Hauptteils als auch an der Außenseite der vorderen Seitenabschnitte lösbar festlegbar sind, wobei die Haltekräfte zwischen den Verschlussmitteln und der Außenseite der vorderen Seitenabschnitte vorzugsweise größer sind als die Haltekräfte zwischen den Verschlussmitteln und der Außenseite des Hauptteils. Dies veranlasst den Benutzer in der Mehrzahl der Fälle, die Verschlussmittel an den vorderen Seitenabschnitten festzulegen. Die Haltekräfte als Über-Bauch-Haltekräfte ermittelt zwischen den insbesondere mechanischen Verschlusshilfen aufweisenden Verschlussmitteln und der Außenseite des Hauptteils betragen vorzugsweise 20-57 N/25mm, insbesondere 25-50 N/25mm. Des Weiteren betragen die Über-Bauch-Haltekräfte zwischen den Verschlussmitteln und der Außenseite der Seitenteile im Vorderbereich vorzugsweise 58-90 N/mm, insbesondere 60-80 N/25mm. Des Weiteren erweist es sich als vorteilhaft, wenn die Über-Bauch-Haltekräfte zwischen den Verschlussmitteln und der Außenseite der hinteren Seitenabschnitte geringer sind als die Über-Bauch-Haltekräfte zwischen den Verschlussmitteln und der Außenseite der vorderen Seitenabschnitte. Auch dies veranlasst den Benutzer in der Mehrzahl der Fälle, die Verschlussmittel an den vorderen Seitenabschnitten festzulegen. Im Rahmen der vorliegenden Erfindung wurden die Über-Bauch-Haltekräfte nach der in WO2008049546A1 beschriebenen Prüfmethode ermittelt.

Die Außenseite des Hauptteils der Inkontinenzwegwerfwindel wird vorzugsweise zumindest bereichsweise, insbesondere aber vollflächig durch einen Vliesstoff gebildet. Dies vermittelt der Inkontinenzwegwerfwindel einen "textile-like" Eindruck. Solchenfalls ist es vorteilhaft, das Backsheet des Hauptteils aus einem Vliesfolienlaminat zu bilden, wobei die Vlieslage außen und die Folienlage innen zum Saugkörper gerichtet zu liegen kommt, so dass die Vlieslage die Außenseite des Hauptteils bildet. Damit ist zum einen die Flüssigkeitsundurchlässigkeit des Hauptteils sichergestellt und zum anderen der hautfreundliche Charakter der Windel gesichert. Die Folienlage dieses Vliesfolienlaminates ist dann vorzugsweise aus einer ein- oder mehrschichtigen flüssigkeitsundurchlässigen, vorzugsweise aber gleichwohl atmungsaktiven Folie gebildet, wobei die Atmungsaktivität der vorderen und/oder der hinteren Seitenabschnitte vorzugsweise größer ist als die Atmungsaktivität des das Backsheet der Inkontinenzwegwerfwindel bildenden Vliesfolienlaminates.

Vorteilhaft unterscheiden sich die hinteren Seitenabschnitte von den vorderen Seitenabschnitten bezüglich mindestens einer, insbesondere mindestens zweier, weiter insbesondere mindestens dreier und weiter insbesondere mindestens vierer ihrer Primäreigenschaften ausgewählt aus der Gruppe Art des Materials, Flächengewicht, Atmungsaktivität, Dichte, Dehnbarkeit, Verschlusskraft, Flächenerstreckung, Dicke, Farbe. Diesbezüglich wird hiermit ausdrücklich auf den Offenbarungsgehalt der WO2009/015746 Bezug genommen.

In Weiterbildung der Erfindung erweist es sich als vorteilhaft, wenn die Länge der vorderen und/oder hinteren Seitenabschnitte, also deren maximale Erstreckung in Windellängsrichtung mindestens 10 cm, insbesondere mindestens 15 cm, weiter insbesondere mindestens 18 cm, weiter insbesondere mindestens 22 cm und weiter insbesondere höchstens 45 cm beträgt. Vorteilhafterweise beträgt die Gesamtlänge der Inkontinenzwegwerfwindel 50-120 cm, insbesondere 60-110 cm und weiter insbesondere 70-110 cm. In Weiterbildung der Erfindung erweist es sich als vorteilhaft, wenn die Breite der vorderen und/oder hinteren Seitenabschnitte, also die maximale Erstreckung der Seitenabschnitte über den Seitenrand des Windelhauptteils hinaus 10-40 cm, insbesondere 12-30 cm, weiter insbesondere 13-25 cm beträgt. Vorzugsweise weisen die vorderen Seitenabschnitte die gleiche Breite auf wie die hinteren Seitenabschnitte.

Die Erfindung betrifft außerdem ein Verfahren zum Herstellen einer Inkontinenzwegwerfwindel, wobei zur Konturierung der beidseitigen Beinöffnungsbereiche der Inkontinenzwegwerfwindel beidseits je ein kontinuierlich oder quasikontinuierlich geführter, den hinteren mit Verschlussmitteln bereits versehenen Seitenabschnitt, den Hauptteil und den vorderen Seitenabschnitt erfassender Abtrennvorgang ausgeführt wird, so dass von hinterem Seitenabschnitt, Hauptteil und vorderem Seitenabschnitt ein zusammenhängender Schnittabfall gebildet wird, der abgeführt werden muss, und wodurch der Abstand C der Verschlussmittel vom unteren Rand der hinteren Seitenabschnitte resultiert.

In Weiterbildung dieses Verfahrens ist vorgesehen, dass der Schnittabfall von einer Transferwalze mit von ihrer Oberfläche vorstehenden stift-, noppen-, haken- oder widerhakenförmigen mechanischen Elementen ergriffen und abgeführt wird.

Vorzugsweise wird zum Ergreifen des Schnittabfalls außerdem eine Unterdruckunterstützung bei der Transferwalze verwendet.

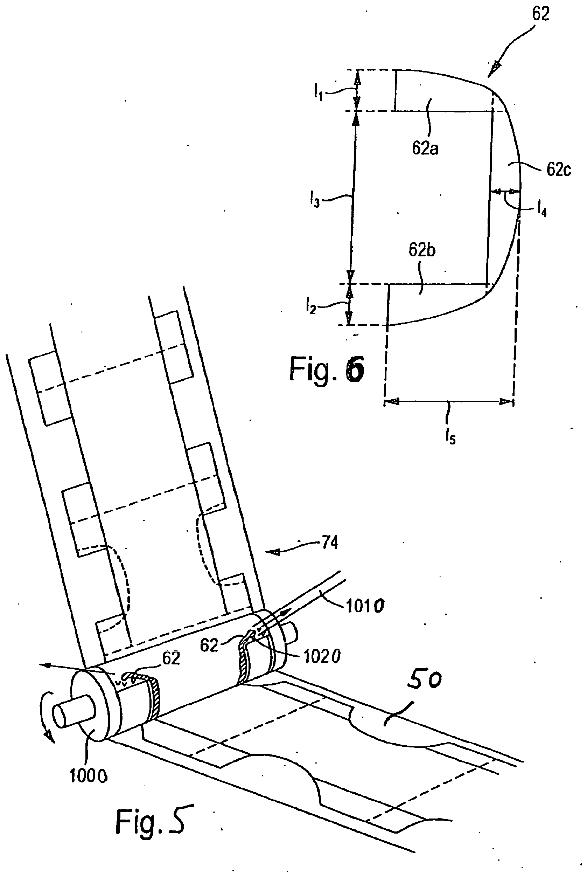

Das erfindungsgemäße Verfahren wird vorteilhaft außerdem dadurch weitergebildet, dass die Erstreckung I₅ des Schnittabfalls (vgl. Figur 6) in der Querrichtung 150mm-350mm, insbesondere 190 mm-300mm beträgt. Vorzugsweise beträgt die Erstreckung I₃ des vom Hauptteil abgetrennten Bereichs des Schnittabfalls in der Längsrichtung 110 bis 500 mm, insbesondere 200 bis 450 mm. Weiter vorzugsweise beträgt die Quererstreckung I₄ des vom Hauptteil abgetrennten Bereichs des Schnittabfalls 5 bis 100 mm, insbesondere 8 bis 70 mm, weiter insbesondere 10 bis 60 mm. Weiter vorzugsweise beträgt die größte Längserstreckung I₁, I₂ des vom hinteren und/oder vorderen Seitenabschnitt abgetrennten Bereichs 20 bis 180 mm insbesondere 30 bis 100mm.

In der Zeichnung zeigen
Figur 1 eine Draufsicht auf einen Inkontinenzartikel in schematischer Darstellung mit beidseits angefügten Seitenabschnitten;
Figuren 2a und 2b je eine vergrößerte Teilansicht der Inkontinenzwegwerfwindel nach Figur 1;
Figur 3 beispielhaft eine weitere erfindungsgemäße Seitenabschnittsgeometrie;

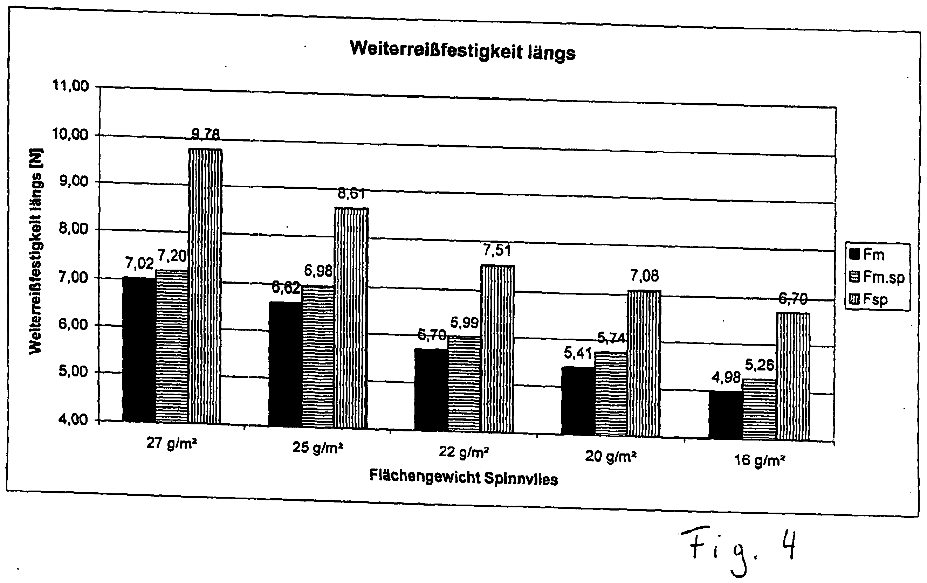

Figur 4 eine grafische Darstellung von ermittelten Weiterreißfestigkeiten;
Figur 5 eine perspektivische Ansicht der Bahnführung über eine Transferwalze mit vorstehenden mechanischen Elementen zum Abführen des Schnittabfalls;
Figur 6 eine Darstellung eines abgetrennten Schnittabfalls;
Figuren 7 und 8 eine Darstellung der Prüfung der Seitenabschnittsausreißfestigkeit.

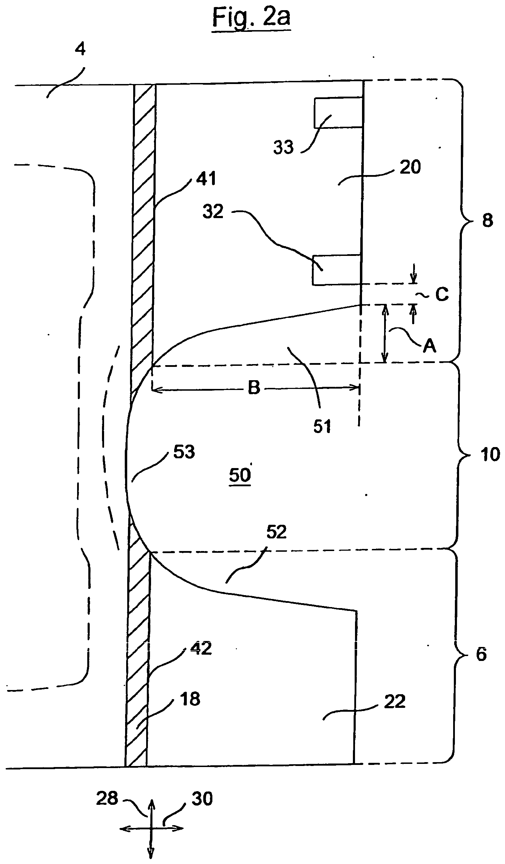

Figur 1 zeigt schematisch, nicht maßstabsgerecht eine Draufsicht auf die Innenseite, also die körperzugewandte Seite einer absorbierenden Inkontinenzwegwerfwindel 2 in eben ausgefaltetem Zustand. Die Inkontinenzwegwerfwindel 2 umfasst einen Hauptteil 4 mit einem Vorderbereich 6, einem Rückenbereich 8 und einem in Längsrichtung dazwischen liegenden Schrittbereich 10. Außerdem angedeutet ist ein Saugkörper 12, der überlicherweise zwischen chassisbildenden Materialien des Hauptteils 4, also insbesondere zwischen einem flüssigkeitsdurchlässigen aus einem Vliesstoffmaterial gebildeten Topsheet 11 und einem im Wesentlichen flüssigkeitsundurchlässigen aus einem Folienmaterial gebildeten Backsheet 13 des Hauptteils 4 angeordnet ist. Das Backsheet 13 kann auch aus einem flüssigkeitsundurchlässigem Vliesstoff oder einem Vliesfolienlaminat gebildet sein, wobei die Vlieslage dann außen und die Folienlage innen zum Saugkörper gerichtet zu liegen kommt. Dies vermittelt der Inkontinenzwegwerfwindel 2 einen "textile-like" Eindruck. Seitlich neben den Längsrändern des Saugkörpers 12 sind erste elastische Elemente 80 an den Hauptteil 4, zwischen Topsheet 11 und Backsheet 13 angefügt. Die elastischen Elemente 80 verlaufen im Wesentlichen in der Längsrichtung, also mit einer wesentlichen Komponente in Längsrichtung, wobei sie einen gekrümmten Verlauf entlang des dem Schrittbereich 10 zuzuordnenden Beinöffnungsbereichsabschnittes nehmen. Die Inkontinenzwegwerfwindel 2 umfasst des Weiteren vordere Seitenabschnitte 22 und hintere Seitenabschnitte 20, die als separate Vliesstoffkomponenten beidseits an den Hauptteil 4 angefügt sind. Wie eine vergrößerte nicht maßstabsgerechte Darstellung einer Teilansicht der Figur 1 zeigt (Figur 2a) sind die Seitenabschnitte 20, 22 in einem schraffiert dargestellten Überlappungsbereich 18 mit chassisbildenden Materialien des Hauptteils 4, also beispielsweise mit dem Backsheet 13 und/oder dem Topsheet 11 unlösbar verbunden. Die Seitenabschnitte 20, 22 erstrecken sich über die vorderen und hinteren seitlichen Längsränder 42, 41 des Hauptteils in Querrichtung 30 hinaus.

Unter vorderen und hinteren seitlichen Längsrändern 42, 41 des Hauptteils werden im Rahmen der vorliegenden Erfindung diejenigen Längsrandbereiche des Hauptteils verstanden, an die die Seitenabschnitte angefügt sind und über welche diese sich hinaus erstrecken.

Die Längserstreckung der vorderen und hinteren Seitenränder des Hauptteils 42, 41 definieren damit auch die Längserstreckung des Vorderbereiches 6 und des Rückenbereiches 8 der Inkontinenzwegwerfwindel 2.

Es wird hiermit außerdem klargestellt, dass die Begriffe "Seitenrand" und "seitlicher Längsrand" vor- und nachstehend synonym verwendet werden.

Die Seitenabschnitte 20, 22 sind dafür gedacht und bestimmt, im angelegten Zustand der Inkontinenzwegwerfwindel 2 miteinander verbunden zu werden, um einen in Umfangsrichtung durchgehenden Hüftbereich des Hygieneartikels zu bilden. Dabei werden jeweils die auf einer Seite des Hauptteils 4 vorgesehenen Seitenabschnitte 20, 22 miteinander verbunden. Hierzu sind an den hinteren Seitenabschnitten 20 beinöffnungsnahe mechanische Verschlussmittel 32 und beinöffnungsferne Verschlussmittel 33, insbesondere mit mechanischen Verschlusshilfen wie Kletthaken, vorgesehen, welche auf der Außenseite der vorderen und hinteren Seitenabschnitte 20, 22 lösbar festlegbar sind. Vorzugsweise sind die Verschlussmittel außerdem auf der Außenseite des Hauptteils lösbar festlegbar. Sowohl die vorderen Seitenabschnitte 22 als auch die hinteren Seitenabschnitte 20 sind aus einem Vliesstoffmaterial, im dargestellten Fall aus einem PP-Spinnvlies, Pegatex S, Hersteller: Pegas a.s.,Primetická 86, 66904 Znojmo, CZ, gebildet. Das Flächengewicht des Vliesstoffmaterials der vorderen Seitenabschnitte beträgt 30 g/m². Die Faserstärke der das Vliesstoffmaterial bildenden Fasern beträgt 2 dtex. Die Über-Bauch-Haltekräfte zwischen den Verschlussmitteln 32 und der Außenseite der vorderen Seitenabschnitte 22 betragen vorzugsweise mindestens 58 N/25mm.

Das Flächengewicht des Vliesstoffmaterials der hinteren Seitenabschnitte 20 beträgt im dargestellten Fall 27 g/m².

Die Weiterreißfestigkeiten des Vliesmaterials der hinteren Seitenabschnitte, gemessen in der Längsrichtung 28 beträgt:
Fm: 7,0 N
Fm.sp: 7,2 N
Fsp: 9,8 N.

Die Über-Bauch-Haltekräfte zwischen den Verschlussmitteln 32, 33 und der Außenseite der hinteren Seitenabschnitte 20 sind geringer als die Über-Bauch-Haltekräfte zwischen den Verschlussmitteln 32, 33 und der Außenseite der vorderen Seitenabschnitte 22.

Wie aus Figur 1 erkennbar ist, weisen die hinteren Seitenabschnitte 20 eine größere Flächenerstreckung auf als die vorderen Seitenabschnitte 22.

Die vorderen und hinteren Seitenabschnitte unterscheiden sich damit in zumindest drei ihrer Primäreigenschaften, nämlich dem Flächengewicht, der Verschlusskraft und der Flächenerstreckung.

Der Unterschied in der Verschlusskraft zwischen den vorderen und hinteren Seitenabschnitten veranlasst den Benutzer, die Verschlussmittel 32, 33 bevorzugt an den vorderen Seitenabschnitten 22 festzulegen, was der Passform der Windel förderlich ist. Wie Figur 1 weiter zu erkennen gibt, sind die Beinöffnungsbereiche 50 durch zum Schrittbereich hin kurvenförmig ausgebildete vordere und hintere Seitenabschnitte 20, 22, welche die Seitenabschnittsbeinöffnungsbereiche bilden, sowie durch die sanduhrförmige Konturierung des Hauptteils 4 gebildet. Unter einer sanduhrförmigen Konturierung des Hauptteils wird hier jede Form der Verengung des Hauptteils 4 im Schrittbereich 10 verstanden, also jede, auch jede nicht oder nicht ausschließlich kurvenförmige Form, bei der der Schrittbereich 10 des Hauptteils 4 eine geringere Erstreckung in Querrichtung 30 aufweist als Vorderbereich 6 und/oder Rückenbereich 8 des Hauptteils.

Die Stetigkeit der Beinöffnung 50 ist gebildet durch je, das heißt auf jeder Seite, einen einzigen Schnitt, welcher sowohl die Seitenabschnitte 20, 22 als auch den Hauptteil 4 erfasst und hierbei stetig durch zu trennendes Seitenrand- oder Hauptteilmaterial hindurchgeführt ist. Die Beinöffnung 50 umfasst somit einen vorderen Seitenabschnittsbeinöffnungsbereich 52, einen hinteren Seitenabschnittsbeinöffnungsbereich 51 und den Hauptteilbeinöffnungsbereich 53 (Figur 2a).

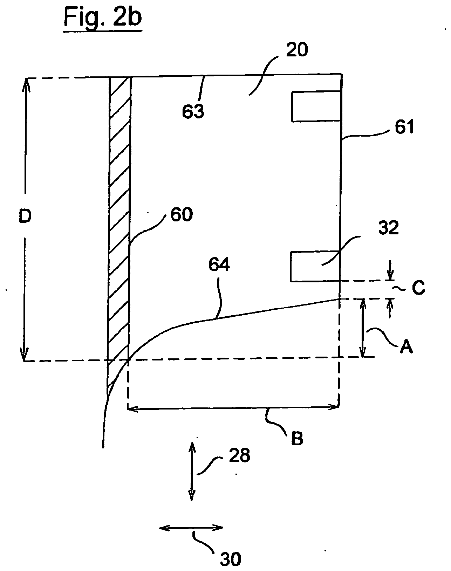

In der Figur 2a ist außerdem die Positionierung der beinöffnungsnahen Verschlussmittel 32 mit dem Abstand C sowie das Längen-zu-Breitenverhältnis R=A/B illustriert. Um die Figur 2a nicht zu überfrachten, ist in Figur 2b der hintere Seitenabschnitt 20 mit den für die Ermittlung der erfindungsgemäßen Parameter erforderlichen Abständen A, B und C nochmals dargestellt: Der hinterer Seitenabschnitt 20 ist begrenzt durch einen inneren Rand 60, welcher dem hinteren Seitenrand 41 des Hauptteils entspricht, durch einen äußeren Rand 61, sowie einem oberen Rand 63 und einem unteren Rand 64, welcher die Kontur des Seitenabschnittsbeinöffnungsbereiches bildet. Oberer Rand 63 und unterer Rand 64 verbinden den inneren Rand 60 mit dem äußeren Rand 61. Vorzugsweise verlaufen innerer Rand 60 und äußerer Rand 61 parallel zueinander, weiter bevorzugt verlaufen innerer 60 und/oder äußerer Rand 61 zumindest abschnittsweise parallel zu einer Längsrichtung 28 der Inkontinenzwegwerfwindel. Vorzugsweise weist der innere Rand 60 eine größere Erstreckung D in der Längsrichtung 28 auf als der äußere Rand 61.

Abstand A ist im Rahmen der vorliegenden Erfindung definiert als die größte Erstreckung des unteren Randes 64 in der Längsrichtung 28. Abstand B ist definiert als die größte Erstreckung des hinteren Seitenabschnitts in der Querrichtung 30, also in Querrichtung die größte Distanz zwischen innerem Rand 60 und äußerem Rand 61, mithin die Seitenabschnittsbreite. Abstand C ist definiert als die in der Längsrichtung 28 zu ermittelnde kürzeste Distanz zwischen einem beinöffnungsnahen Verschlussmittel 32 und dem unteren Rand 64.

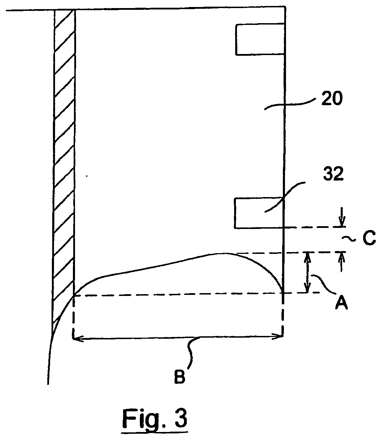

Figur 3 zeigt eine alternative erfindungsgemäße Seitenabschnittgeometrie, bei der die größte Erstreckung A des unteren Randes 64 in der Längsrichtung 28 zwischen dem äußeren Rand 61 und dem inneren Rand 60 zu messen ist, da die Kontur des unteren Randes 64 ein echtes Maximum aufweist.

A beträgt im dargestellten Fall der Figuren 2a, 2b 55 mm, B beträgt 225 mm, R beträgt somit 0,24. Die Länge D des inneren Randes 50 beträgt 350 mm. Das beinöffnungsnahe Verschlussmittel 32 ist sehr dicht, im Abstand C = 16 mm von dem unteren Rand 54 des hinteren Seitenabschnitts herstellerseitig befestigt.

Die Ausreißfestigkeit der hinteren Seitenabschnitte beträgt 64,5 N. Zum Vergleich wurde die Seitenabschnittsausreißfestigkeit einer Vergleichswindel ermittelt, die aus identischen Materialien gefertigt wurde und eine identische maximale Seitenabschnittslänge und -Breite aufwies, jedoch mit Seitenabschnitten rechteckiger Kontur (so wie schematisch dargestellt in WO2005102241) ausgestattet war. Lediglich das Flächengewicht des PP-Spinnvlieses (Hersteller: Pegas a.s.) der hinteren Seitenabschnitte war mit 30 g/m² geringfügig, das heißt um 3 g/m² schwerer als das der Seitenabschnitte der oben beschriebenen erfindungsgemäßen Windel. Die Seitenabschnittsausreißfestigkeit dieser Vergleichswindel betrug lediglich 38,8 N. Dies zeigt, dass bereits eine geringfügige Konturierung, ausgedrückt durch einen geringen R-Wert, die Seitenabschnittausreißfestigkeit deutlich positiv beeinflusst.

Im Rahmen der vorliegenden Erfindung sind und werden die Seitenabschnittausreißfestigkeiten mittels des weiter unten erläuterten Prüfverfahrens gemessen.

Im Rahmen der vorliegenden Erfindung sind und werden die Weiterreißfestigkeiten als Weiterreißkraft nach der In DIN EN ISO 13937-2 spezifizierten Prüfmethode ermittelt. Abweichend davon beträgt die Probenlänge 150 mm. Der mittige Einschnitt weist eine tiefe von 50mm auf. Die Verformungsgeschwindigkeit wird auf 200 mm/min eingestellt. Die Auswertung erfolgt mittels einer elektronischen Einrichtung. Abweichend von DIN EN ISO 13937-2 ist ein auszuwertender Spitzenwert durch einen Kraftanstieg oder einen Kraftabfall von mindestens 0,2 N gekennzeichnet. Es werden somit obere und untere Kraftspitzen bei Ermittlung des Fm.sp berücksichtigt. Neben dem arithmetischen Mittelwert der Kraftspitzen F m,sp, werden außerdem der Maximalwert aller oberen Kraftspitzen eines jeweiligen Prüflings Fsp und die über die gesamte auszuwertende Kraftverlaufskurve berechnete mittlere Kraft Fm ermittelt.

Figur 3 zeigt eine grafische Darstellung ermittelter Weiterreißfestigkeiten von Seitenabschnitten bildenden Spinnvliesmaterialien, die sich lediglich hinsichtlich des Flächengewichts voneinander unterscheiden. Die Seitenabschnittsausreißfestigkeiten daraus hergestellter erfindungsgemäßer Inkontinenzwegwerfwindeln sinken mit abfallender Weiterreißfestigkeit der Seitenabschnittsmaterialien. Mit vorliegender Erfindung wurde aber erkannt, dass auch das getestete 16g/m²-Spinnvlies mit einer Weiterreißfestigkeit von Fm 4,48 in Kombination mit der erfindungsgemäßen Konturierung der Seitenabschnitte den Anforderungen an die Ausreißfestigkeit der Seitenabschnitte in Gebrauch der Windel genügt, selbst wenn die Verschlussmittel weniger als 5 cm vom unteren Rand der Seitenabschnitte beabstandet herstellerseitig befestigt sind. Es wird ausdrücklich darauf hingewiesen, dass die Vorsehung eines Flächengewichtes von mindestens 16 g/m² keinesfalls eine hinreichende Bedingung dafür ist, die geforderten Weiterreißfestigkeiten sicherzustellen. Die Änderung des Flächengewichts des Vliesmaterials ist hingegen die einfachste Methode um bei ansonsten unveränderten Vliesstoffcharakteristika die Weiterreißfestigkeit zu variieren. Die Art des Vliesbildungsprozesses, die verwendeten Polymere das ggf. eingesetzte Bindemittel sind nur einige weitere dem Fachmann an sich geläufige Möglichkeiten, die Festigkeiten des Vliesstoffes zu beeinflussen.

Figur 5 zeigt schematisch einen Ausschnitt des erfindungsgemäßen Verfahrens zur Herstellung der Inkontinenzwegwerfwindel. Die Bahnführung erfolgt über eine Transferwalze 1000 zum Abführen des Schnittabfalls 62, die den an der Stelle 74 nicht dargestellten Messerwalzen zur Konturierung der beidseitigen Beinöffnungsbereiche 50 nachgeordnet ist und mittels derer der zusammenhängende Schnittabfall 62 aus hinterem Seitenabschnitt, Hauptteil und vorderem Seitenabschnitt aus dem Prozess abgeführt werden kann. Hierzu weist die Transferwalze 1000 zum Ergreifen des Schnittabfalls 62 zoniert angeordnete stiftförmige mechanische Elemente 1020 auf. Der Schnittabfalls kann insbesondere nach dem Ergreifen durch die Transferwalze 1000 mittels einer nur angedeuteten Saugeinrichtung 1010 abgesaugt werden. Hinsichtlich der detaillierten Ausbildung der Transferwalze wird vollumfänglich auf die DE102008056220 verwiesen.

Figur 6 zeigt schematisch, nicht maßstabsgerecht, die Erstreckung I₅ des Schnittabfalls 62 in der Querrichtung von vorzugsweise 150mm-350mm, insbesondere 190 mm-300mm. Vorzugsweise beträgt die Erstreckung I₃ des vom Hauptteil abgetrennten Bereichs 62c des Schnittabfalls in der Längsrichtung 110 bis 500 mm, insbesondere 200 bis 450 mm. Weiter vorzugsweise beträgt die Quererstreckung I₄ des vom Hauptteil abgetrennten Bereichs 62c des Schnittabfalls 5 bis 100 mm, insbesondere 8 bis 70 mm, weiter insbesondere 10 bis 60 mm. Weiter vorzugsweise beträgt die größte Längserstreckung I₁, I₂ des vom hinteren und/oder vorderen Seitenabschnitt abgetrennten Bereichs 62a, 62b 20 bis 180 mm insbesondere 30 bis 100mm.

Mit der vorliegenden Erfindung ist es somit gelungen, erstmals eine Inkontinenzwegwerfwindel mit an den Hauptteil angefügten vorderen und hinteren Seitenabschnitten bereitzustellen, welche eine in Gebrauch der Windel ausreichende Seitenabschnittsausreißfestigkeit aufweist und zugleich den Anlege- und Tragekomfort der Inkontinenzwegwerfwindel ausreichend berücksichtigt.

### Prüfmethode Seitenabschnittsausreißfestigkeit

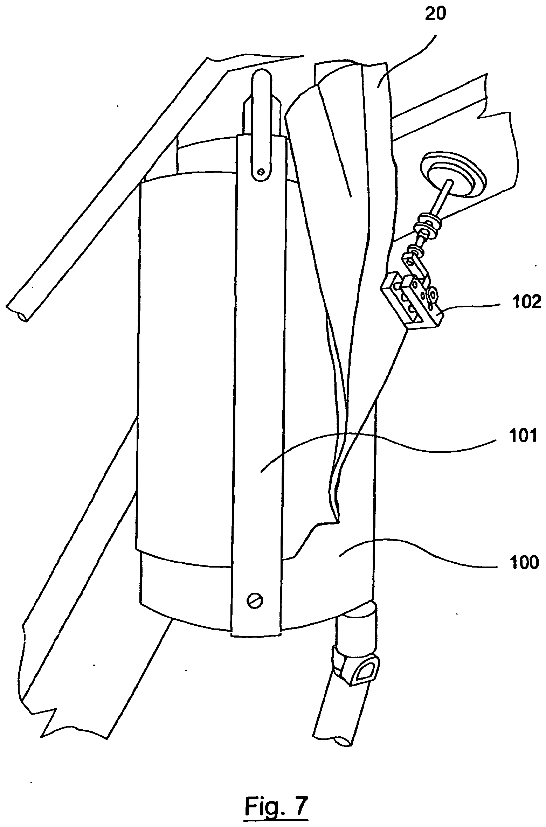

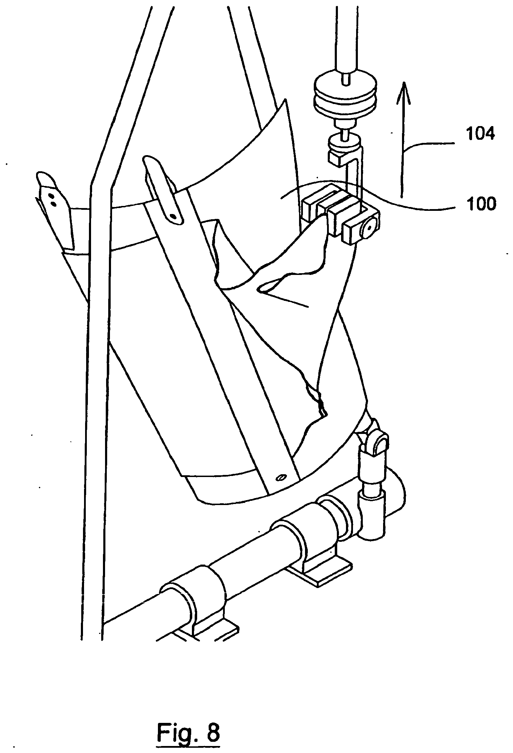

Eine Inkontinenzwegwerfwindel wird 60 cm unterhalb des hinteren Seitenabschnittes unter Zerstörung des Hauptteils in Querrichtung getrennt (geschnitten oder gestanzt). Der den unteren Seitenabschnitt enthaltende Prüfling wird in das Zugprüfgerät eingespannt (siehe Figuren 7 und 8).

Der Prüfling wird dabei mit seiner Innenseite anliegend an und über eine gekrümmte Fläche 100 (Radius der Krümmung 19 cm) gelegt, welche die Rundung des Rückenbereichs eines Benutzers simulieren soll und in die Vorrichtung des Zugprüfgeräts eingespannt (siehe Figuren 7 und 8). Dargestellt ist die Prüfung eines rechten hinteren Seitenabschnitts. Im Falle der Prüfung eines linken hinteren Seitenabschnittes ist eine spiegelbildlich angeordnete Prüfvorrichtung und -anordnung vorgesehen.

Das Produkt soll dergestalt in die Vorrichtung eingespannt werden, dass der Prüfling durch die feststehende Klemme 101 über die gesamte Länge des Prüflings im Abstand (Querrichtung) von 60mm vom inneren Rand des Seitenabschnitts 20 fixiert wird. Die bewegbare Klemme 102 wird am unteren Ende des äußeren Randes des Seitenabschnittes 20 über eine Länge von 60mm und eine Breite von 30mm fixiert. Die gekrümmte Fläche 100 ist nach vorn gekippt (geneigt), so dass der Seitenabschnitt beim nachfolgenden Zugversuch an die gekrümmte Fläche anliegen kann (siehe Figur 8). Durch gesteuerte Bewegungen der bewegbaren Klemme 102 in Richtung des Pfeils 104 wird ein Zugprüfversuch durchgeführt. Die Prüfgeschwindigkeit, mit der die bewegbare Klemme 102 mechanisch vom Hauptteil des Inkontinenzproduktes weg bewegt wird, beträgt 1800 mm/min. Dabei wird eine Vorkraft von 0,2 N (Newton) aufgewendet. Das Verfahren wird beendet, sobald der Seitenabschnitt über eine Länge von mindestens 5 cm einreißt. Insgesamt sollen eine Mindestanzahl von n = 5 Prüfungen durchgeführt werden. Zur Auswertung werden die gemessenen Maximalkräfte in [N] eines jeden Zugversuches gemittelt.

## Patentansprüche

1. Absorbierende Inkontinenzwegwerfwindel (2) des offenen Typs, mit einem Hauptteil (4), umfassend einen Vorderbereich (6) mit vorderen seitlichen Längsrändern (42), einen Rückenbereich (8) mit hinteren seitlichen Längsrändern (41) und einen in Längsrichtung (28) dazwischen liegenden, zwischen den Beinen eines Benutzers zu liegen kommenden Schrittbereich (10), wobei der Hauptteil (4) einen Saugkörper (12) umfasst, und mit beidseits an den Rückenbereich (8) angefügten hinteren Seitenabschnitten (20) und beidseits an den Vorderbereich (6) angefügten vorderen Seitenabschnitten (22), welche sich in Querrichtung (30) über die seitlichen vorderen und hinteren Längsränder (42, 41) des Hauptteils (4) hinaus erstrecken, und wobei die hinteren Seitenabschnitte (20) erste beinöffnungsnahe Verschlussmittel (32) mit Verschlusshilfen aufweisen und wobei die Verschlussmittel (32) zumindest an der Außenseite der vorderen Seitenabschnitte (22) lösbar festlegbar sind und dadurch der Vorderbereich (6) und der Rückenbereich (8) miteinander verbindbar sind, wobei zur Bildung von Seitenabschnittsbeinöffnungsbereichen (51, 52) die Seitenabschnitte (20, 22) zumindest auf der dem Schrittbereich (10) zugewandten Seite schräg zur Längsrichtung (28) verlaufend oder kurvenförmig ausgebildet sind und wobei ein äußerer Rand (61) der hinteren Seitenabschnitte (20) parallel zur Längsrichtung (28) verläuft, **dadurch gekennzeichnet, dass** das Längen-zu-Breitenverhältnis R des Seitenabschnittsbeinöffnungsbereiches (51) der hinteren Seitenabschnitte (20) 0,1-0,4 beträgt, dass die Weiterreißfestigkeit Fm des die hinteren Seitenabschnitte bildenden Materials in Windellängsrichtung mindestens 4,0 N beträgt und dass der Abstand C der beinöffnungsnahen Verschlussmittel (32) von dem unteren Rand der hinteren Seitenabschnitte höchsten 5 cm beträgt.

2. Absorbierende Inkontinenzwegwerfwindel (2) nach Anspruch 1, **dadurch gekennzeichnet, dass** das Längen-zu-Breitenverhältnis R des Seitenabschnittsbeinöffnungsbereiches der hinteren Seitenabschnitte (20) mindestens 0,15, insbesondere 0,18-0,35 und weiter insbesondere 0,20-0,32 beträgt.

3. Absorbierende Inkontinenzwegwerfwindel (2) nach einem der vorhergehenden Ansprüche, **dadurch gekennzeichnet, dass** der Abstand C der beinöffnungsnahen Verschlussmittel (32) von dem unteren Rand der hinteren Seitenabschnitte höchsten 4 cm, insbesondere höchstens 3,5 cm, weiter insbesondere höchstens 3,0 cm und weiter insbesondere mindestens 0,5 cm beträgt.

4. Absorbierende Inkontinenzwegwerfwindel (2) nach einem der vorhergehenden Ansprüche, **dadurch gekennzeichnet, dass** die hinteren Seitenabschnitte mindestens ein weiteres hüftfernes mit Verschlusshilfen versehenes Verschlussmittel (33) aufweisen.

5. Absorbierende Inkontinenzwegwerfwindel (2) nach einem der vorhergehenden Ansprüche, **dadurch gekennzeichnet, dass** die Weiterreißfestigkeit Fm des die hinteren Seitenabschnitte bildenden Materials in Windellängsrichtung mindestens 5,0 N, insbesondere mindestens 6,0 N, insbesondere mindestens 6,5 N und weiter insbesondere höchstens 10 N beträgt.

6. Absorbierende Inkontinenzwegwerfwindel (2) nach einem der vorhergehenden Ansprüche, **dadurch gekennzeichnet, dass** die Weiterreißfestigkeit Fm.sp des die hinteren Seitenabschnitte bildenden Materials in Windellängsrichtung mindestens 5,5 N, insbesondere mindestens 6,0 N, insbesondere mindestens 6,5 N und weiter insbesondere mindestens 7,0 N und weiter insbesondere höchstens 12 N beträgt.

7. Absorbierende Inkontinenzwegwerfwindel (2) nach einem der vorhergehenden Ansprüche, **dadurch gekennzeichnet, dass** die Weiterreißfestigkeit Fsp des die hinteren Seitenabschnitte bildenden Materials in Windellängsrichtung mindestens 5,5 N, insbesondere mindestens 6,0 N, insbesondere mindestens 6,5 N und weiter insbesondere mindestens 7,0 N und weiter insbesondere höchstens 12 N beträgt.

8. Absorbierende Inkontinenzwegwerfwindel (2) nach einem der vorhergehenden Ansprüche, **dadurch gekennzeichnet, dass** die Seitenabschnitte (20, 22) aus einem Vliesstoffmaterial gebildet sind oder ein Vliesstoffmaterial umfassen.

9. Absorbierende Inkontinenzwegwerfwindel (2) nach einem der vorstehenden Ansprüche, **dadurch gekennzeichnet, dass** die Erstreckung der Seitenabschnitte (20, 22) über den Seitenrand des Windelhauptteils hinaus in Querrichtung 10-40 cm, insbesondere 12-30 cm, weiter insbesondere 13-25 cm beträgt.

10. Absorbierende Inkontinenzwegwerfwindel (2) nach einem der vorstehenden Ansprüche, **dadurch gekennzeichnet, dass** die Länge der hinteren Seitenabschnitte, also deren Erstreckung in Windellängsrichtung mindestens 10 cm, insbesondere mindestens 15 cm, weiter insbesondere mindestens 18 cm und weiter insbesondere mindestens 22 cm und weiter insbesondere höchstens 45 cm beträgt.

11. Verfahren zum Herstellen einer Inkontinenzwegwerfwindel (2) nach einem der vorhergehenden Ansprüche, wobei zur Konturierung von beidseitigen Beinöffnungsbereichen (50) der Inkontinenzwegwerfwindel (2) beidseits je ein kontinuierlich oder quasikontinuierlich geführter, den hinteren mit Verschlussmitteln (32) bereits versehenen Seitenabschnitt (20), den Hauptteil (4) und den vorderen Seitenabschnitt (22) erfassender Abtrennvorgang ausgeführt wird, so dass von hinterem Seitenabschnitt (20), Hauptteil (4) und vorderem Seitenabschnitt (22) ein zusammenhängender Schnittabfall (62) gebildet wird, der abgeführt werden muss, und wodurch der Abstand C der beinöffnungsnahen Verschlussmittel (32) vom unteren Rand der hinteren Seitenabschnitte (20) resultiert.

12. Verfahren nach Anspruch 11, **dadurch gekennzeichnet, dass** der Schnittabfall (62) von einer Transferwalze (1000) mit von ihrer Oberfläche vorstehenden stift-, noppen-, haken- oder widerhakenförmigen mechanischen Elementen (1020) ergriffen und abgeführt wird.

13. Verfahren nach Anspruch 11 oder 12, **dadurch gekennzeichnet, dass** zum Ergreifen des Schnittabfalls (62) eine Unterdruckunterstützung bei der Transferwalze (1000) verwendet wird.

14. Verfahren nach Anspruch 11, 12 oder 13, **dadurch gekennzeichnet, dass** die Erstreckung l₅ des Schnittabfall (62) in der Querrichtung 150mm-350mm, insbesondere 190 mm-300mm beträgt, dass die Erstreckung l₃ des vom Hauptteil abgetrennten Bereichs 62c des Schnittabfalls in der Längsrichtung 110 bis 500 mm insbesondere 200 bis 450 mm beträgt, dass die Quererstreckung l₄ des vom Hauptteil abgetrennten Bereichs 62c des Schnittabfalls 5 bis 100 mm insbesondere 8 bis 70 mm, weiter insbesondere 10 bis 60 mm beträgt und dass die größte Längserstreckung l₁, l₂ des vom hinteren und/oder vorderen Seitenabschnitt abgetrennten Bereichs 62a, 62b 20 bis 180 mm insbesondere 30 bis 100mm beträgt.

## Claims

1. Disposable absorbent incontinence diaper (2) of the open type, having a main part (4), comprising a front region (6) having front lateral longitudinal edges (42), a rear region (8) having rear lateral longitudinal edges (41) and a crotch region (10) that is located in between in the longitudinal direction (28) and comes to lie between the legs of a user, wherein the main part (4) comprises an absorbent pad (12), and having rear side portions (20) that are attached to the rear region (8) on both sides and front side portions (22) that are attached to the front region (6) on both sides, said rear and front side portions extending in the transverse direction (30) beyond the lateral front and rear longitudinal edges (42, 41) of the main part (4), and wherein the rear side portions (20) have first closure means (32) which are close to the leg opening and have closure aids and wherein the closure means (32) can be secured in a detachable manner at least on the outer side of the front side portions (22), as a result of which the front region (6) and the rear region (8) can be connected together, wherein, in order to form side-portion leg-opening regions (51, 52), the side portions (20, 22) are formed, at least on the side facing the crotch region (10), in a manner extending obliquely with respect to the longitudinal direction (28) or in a curved manner, and wherein an outer edge (61) of the rear side portions (20) extends parallel to the longitudinal direction (28), **characterized in that** the length-to-width ratio R of the side-portion leg-opening region (51) of the rear side portions (20) is 0.1-0.4, **in that** the tear propagation resistance Fm of the material forming the rear side portions is at least 4.0 N in the longitudinal direction of the diaper, and **in that** the spacing C of the closure means (32) close to the leg opening from the lower edge of the rear side portions is at most 5 cm.

2. Disposable absorbent incontinence diaper (2) according to Claim 1, **characterized in that** the length-to-width ratio R of the side-portion leg-opening region of the rear side portions (20) is at least 0.15, particularly 0.18-0.35 and more particularly 0.20-0.32.

3. Disposable absorbent incontinence diaper (2) according to either of the preceding claims, **characterized in that** the spacing C of the closure means (32) close to the leg opening from the lower edge of the rear side portions is at most 4 cm, particularly at most 3.5 cm, more particularly at most 3.0 cm and more particularly at least 0.5 cm.

4. Disposable absorbent incontinence diaper (2) according to one of the preceding claims, **characterized in that** the rear side portions have at least one further closure means (33) which is remote from the hip and is provided with closure aids.

5. Disposable absorbent incontinence diaper (2) according to one of the preceding claims, **characterized in that** the tear propagation resistance Fm of the material forming the rear side portions is at least 5.0 N, particularly at least 6.0 N, particularly at least 6.5 N and more particularly at most 10 N in the longitudinal direction of the diaper.

6. Disposable absorbent incontinence diaper (2) according to one of the preceding claims, **characterized in that** the tear propagation resistance Fm.sp of the material forming the rear side portions is at least 5.5 N, particularly at least 6.0 N, particularly at least 6.5 N and more particularly at least 7.0 N and more particularly at most 12 N in the longitudinal direction of the diaper.

7. Disposable absorbent incontinence diaper (2) according to one of the preceding claims, **characterized in that** the tear propagation resistance Fsp of the material forming the rear side portions is at least 5.5 N, particularly at least 6.0 N, particularly at least 6.5 N and more particularly at least 7.0 N and more particularly at most 12 N in the longitudinal direction of the diaper.

8. Disposable absorbent incontinence diaper (2) according to one of the preceding claims, **characterized in that** the side portions (20, 22) are formed from a nonwoven material or comprise a nonwoven material.

9. Disposable absorbent incontinence diaper (2) according to one of the preceding claims, **characterized in that** the extent of the side portions (20, 22) beyond the lateral edge of the main part of the diaper is 10-40 cm, particularly 12-30 cm, more particularly 13-25 cm in the transverse direction.

10. Disposable absorbent incontinence diaper (2) according to one of the preceding claims, **characterized in that** the length of the rear side portions, that is the extent thereof in the longitudinal direction of the diaper, is at least 10 cm, particularly at least 15 cm, more particularly at least 18 cm and more particularly at least 22 cm and more particularly at most 45 cm.

11. Method for producing a disposable incontinence diaper (2) according to one of the preceding claims, wherein, in order to contour leg-opening regions (50) on both sides of the disposable incontinence diaper (2), a respective continuously or quasi-continuously guided severing process taking in the rear side portion (20), which is already provided with closure means (32), the main part (4) and the front side portion (22) is executed on each side, so that a coherent offcut (62) which has to be removed is formed from the rear side portion (20), main part (4) and front side portion (22), and as a result of which the spacing C of the closure means (32) close to the leg opening from the lower edge of the rear side portions (20) is produced.

12. Method according to Claim 11, **characterized in that** the offcut (62) is gripped and removed by a transfer roller (1000) having pin-like, nub-like, hook-like or barb-like mechanical elements (1020) that protrude from its surface.

13. Method according to Claim 11 or 12, **characterized in that** in order to grip the offcut (62) use is made of a negative-pressure assistance means at the transfer roller (1000).

14. Method according to Claim 11, 12 or 13, **characterized in that** the extent l₅ of the offcut (62) in the transverse direction is 150 mm-350 mm, particularly 190 mm-300 mm, **in that** the extent l₃ in the longitudinal direction of the region 62c of the offcut severed from the main part is from 110 to 500 mm, particularly from 200 to 450 mm, **in that** the transverse extent l₄ of the region 62c of the offcut severed from the main part is from 5 to 100 mm, particularly from 8 to 70 mm, more particularly from 10 to 60 mm, and **in that** the greatest longitudinal extent l₁, l₂ of the region 62a, 62b severed from the rear and/or front side portion is from 20 to 180 mm, particularly from 30 to 100 mm.

## Revendications

1. Couche pour incontinence absorbante jetable (2) du type ouvert, avec une partie principale (4), comprenant une région avant (6) avec des bords longitudinaux latéraux avant (42), une région arrière (8) avec des bords longitudinaux latéraux arrière (41) et une région de fourche (10) disposée entre elles dans la direction longitudinale (28), venant se placer entre les jambes d'un utilisateur, la partie principale (4) comprenant un corps aspirant (12) et avec des portions latérales arrière (20) réunies de chaque côté à la région arrière (8) et des portions latérales avant (22) réunies de chaque côté à la région avant (6), lesquelles s'étendent dans la direction transversale (30) au-delà des bords longitudinaux latéraux avant et arrière (42, 41) de la partie principale (4), et les portions latérales arrière (20) présentant des premiers moyens de fermeture (32) proches de l'ouverture des jambes avec des auxiliaires de fermeture, et les moyens de fermeture (32) pouvant être fixés de manière amovible au moins sur le côté extérieur des portions latérales avant (22) et de ce fait la région avant (6) et la région arrière (8) pouvant être connectées l'une à l'autre, pour former des régions d'ouverture des jambes de portion latérale (51, 52), les portions latérales (20, 22) étant réalisées au moins du côté tourné vers la région de fourche (10) en s'étendant obliquement par rapport à la direction longitudinale (28) et sous forme courbe et un bord extérieur (61) des portions latérales arrière (20) s'étendant parallèle á la direction longitudinale (28), **caractérisée en ce que** le rapport longueur à largeur R de la région d'ouverture des jambes de portion latérale (51) des portions latérales arrière (20) valant 0,1 à 0,4, **en ce que** la résistance à l'arrachage ultérieur Fm du matériau formant les portions latérales arrière dans la direction longitudinale de la couche étant d'au moins 4,0 N et **en ce que** la distance C des moyens de fermeture (32) proches de l'ouverture des jambes au bord inférieur des portions latérales arrière étant au maximum de 5 cm.

2. Couche pour incontinence absorbante jetable (2) selon la revendication 1, **caractérisée en ce que** le rapport longueur à largeur R de la région d'ouverture des jambes de portion latérale des portions latérales arrière (20) vaut au moins 0,15, en particulier 0,18 à 0,35, et plus particulièrement 0,20 à 0,32.

3. Couche pour incontinence absorbante jetable (2) selon l'une quelconque des revendications précédentes, **caractérisée en ce que** la distance C des moyens de fermeture (32) proches de l'ouverture des jambes au bord inférieur des portions latérales arrière est au maximum de 4 cm, en particulier au maximum de 3,5 cm, et plus particulièrement au maximum de 3,0 cm et encore plus particulièrement d'au moins 0,5 cm.

4. Couche pour incontinence absorbante jetable (2) selon l'une quelconque des revendications précédentes, **caractérisée en ce que** les portions latérales arrière présentent au moins un moyen de fermeture supplémentaire (33) éloigné des hanches, muni d'auxiliaires de fermeture.

5. Couche pour incontinence absorbante jetable (2) selon l'une quelconque des revendications précédentes, **caractérisée en ce que** la résistance à l'arrachage ultérieur Fm du matériau formant les portions latérales arrière dans la direction longitudinale de la couche est d'au moins 5,0 N, en particulier d'au moins 6,0 N, en particulier d'au moins 6,5 N, et en particulier en outre d'au plus 10 N.

6. Couche pour incontinence absorbante jetable (2) selon l'une quelconque des revendications précédentes, **caractérisée en ce que** la résistance à l'arrachage ultérieur Fm.sp du matériau formant les portions latérales arrière dans la direction longitudinale de la couche est d'au moins 5,5 N, en particulier d'au moins 6,0 N, en particulier d'au moins 6,5 N, et en particulier en outre d'au moins 7,0 N et en particulier en outre d'au plus 12 N.

7. Couche pour incontinence absorbante jetable (2) selon l'une quelconque des revendications précédentes, **caractérisée en ce que** la résistance à l'arrachage ultérieur Fsp du matériau formant les portions latérales arrière dans la direction longitudinale de la couche est d'au moins 5,5 N, en particulier d'au moins 6,0 N, en particulier d'au moins 6,5 N et en particulier en outre d'au moins 7,0 N et en particulier en outre d'au plus 12 N.

8. Couche pour incontinence absorbante jetable (2) selon l'une quelconque des revendications précédentes, **caractérisée en ce que** les portions latérales (20, 22) sont formées d'un matériau non tissé ou comprennent un matériau non tissé.

9. Couche pour incontinence absorbante jetable (2) selon l'une quelconque des revendications précédentes, **caractérisée en ce que** l'étendue des portions latérales (20, 22) au-delà du bord latéral de la partie principale de la couche dans la direction transversale est de 10-40 cm, en particulier de 12-30 cm, plus particulièrement de 13-25 cm.

10. Couche pour incontinence absorbante jetable (2) selon l'une quelconque des revendications précédentes, **caractérisée en ce que** la longueur des portions latérales arrière, c'est-à-dire leur étendue dans la direction longitudinale de la couche est d'au moins 10 cm, en particulier d'au moins 15 cm, plus particulièrement d'au moins 18 cm et plus particulièrement d'au moins 22 cm et particulièrement en outre d'au plus 45 cm.

11. Procédé de fabrication d'une couche pour incontinence jetable (2) selon l'une quelconque des revendications précédentes, dans lequel pour le profilage de régions d'ouverture de jambe (50) des deux côtés de la couche pour incontinence jetable (2) est réalisée de chaque côté, à chaque fois une opération de sectionnement conduite de manière continue ou quasi-continue, saisissant la portion latérale arrière (20) déjà pourvue de moyens de fermeture (32), la partie principale (4) et la portion latérale avant (22), de telle sorte qu'une chute de découpage (62) rattachée soit formée par la portion latérale arrière (20), la partie principale (4) et la portion latérale avant (22), laquelle doit être évacuée, ce qui produit la distance C des moyens de fermeture (32) proches de l'ouverture de jambe depuis le bord inférieur des portions latérales arrière (20).

12. Procédé selon la revendication 11, **caractérisé en ce que** la chute de découpage (62) est saisie et évacuée par un rouleau de transfert (1000) avec des éléments (1020) mécaniques saillant depuis sa surface, en forme de goupilles, de boutons, de crochets ou de barbes.

13. Procédé selon la revendication 11 ou 12, **caractérisé en ce que** pour saisir la chute de découpage (62), on utilise une assistance par dépression au niveau du rouleau de transfert (1000).

14. Procédé selon la revendication 11, 12 ou 13, **caractérisé en ce que** l'étendue l₅ de la chute de découpage (62) dans la direction transversale vaut 150 mm - 350 mm, en particulier 190 mm - 300 mm, **en ce que** l'étendue l₃ de la région 62c de la chute de découpage sectionnée de la partie principale dans la direction longitudinale vaut 110 à 500 mm, en particulier 200 à 450 mm, **en ce que** l'étendue transversale l₄ de la région 62c de la chute de découpage sectionnée de la partie principale vaut 5 à 100 mm, en particulier 8 à 70 mm, plus particulièrement 10 à 60 mm, et **en ce que** la plus grande étendue longitudinale l₁, l₂ de la région 62a, 62b sectionnée de la portion latérale arrière et/ou avant vaut 20 à 180 mm, en particulier 30 à 100 mm.
